# EUROPÄISCHE PATENTSCHRIFT

(11) **EP 1 963 301 B1**
(45) Veröffentlichungstag und Bekanntmachung des Hinweises auf die Patenterteilung: **02.03.2011**
(21) Anmeldenummer: 06794013.0
(22) Anmeldetag: 11.10.2006
(51) Int. Cl.: C07D 317/36

(54) **VERFAHREN ZUR HERSTELLUNG VON GLYCERINCARBONAT**
PROCESS FOR PREPARING GLYCERYL CARBONATE
PROCEDE DE FABRICATION DE CARBONATE DE GLYCERINE

(30) Priorität: 16.12.2005 DE 102005060732
(43) Veröffentlichungstag der Anmeldung: 03.09.2008
(73) Patentinhaber: Evonik Röhm GmbH, 64293 Darmstadt (DE)
(72) Erfinder: SCHMITT, Bardo, 55252 Mainz (DE); KNEBEL, Joachim, 64665 Alsbach-Hähnlein (DE); CASPARI, Maik, 64665 Alsbach-Hähnlein (DE)
(86) Internationale Anmeldenummer: PCT/EP2006/067279
(87) Internationale Veröffentlichungsnummer: WO 2007/071470

(56) Entgegenhaltungen:
- EP-A- 0 739 888
- EP-A2- 0 478 073
- WO-A-03/042141
- US-A- 2 915 529
- WPI WORLD PATENT INFORMATION DERWENT, DERWENT, GB, Bd. 7, Nr. 95, 29. November 1994 (1994-11-29), XP002008629 & JP 06 329663 A (NISSO MARUZEN CHEM) 29. November 1994 (1994-11-29)

## Beschreibung

Die Erfindung betrifft ein Verfahren zur Herstellung von Glycerincarbonat mit geringem Glyceringehalt.

Glycerincarbonat wird als Zwischenprodukt zur Herstellung von Pharmazeutica und Pestiziden sowie zur Herstellung von (Meth)acrylaten als Vorprodukt für Lackharze verwendet. Eine hohe Reinheit ist deshalb vorteilhaft.

In der US 2915529 wird ein aufwendiges Verfahren zur Herstellung von Glycerincarbonat aus Ethylencarbonat und Glycerin mit NaOH beschrieben. In einem 2-stufigen Verfahren wird der pH-Wert eingestellt, anschließend die Niedersieder abdestilliert und nach einer weiteren Katalysatorzugabe weiter umgesetzt und wiederholt destilliert. Die Angabe der Reinheit von 97,5% enthält auch keine Angaben zur Art der Nebenprodukte. Insbesondere die Anwesenheit von Glycerin bereitet Probleme bei der Weiterverarbeitung, da hiermit bei der Funktionalisierung der Hydroxygruppe, beispielweise durch die Reaktion mit Polyisocyanaten, unerwünschte Nebenreaktion, vorzugsweise Vernetzungsreaktionen bzw. Oligomerisierungen, stattfinden.

Die EP 739888 beschreibt ein Verfahren zur Herstellung von Glycerincarbonat aus Ethylencarbonat und Glycerin mit Amberlyst (Ionentauscher) oder Zeoliten, bei dem mindestens 2-4 % Glycerin im Rohprodukt enthalten sind.

In der JP 2001172277 wird die Herstellung von Glycerincarbonat durch Umesterung von Dimethylcarbonat mit Glycerin unter basischer Katalyse beansprucht. Das Glycerincarbonat hat bei einer Reinheit von 95% einen Glyceringehalt von 1,6%.

In der EP 1423377 wird eine Aufreinigung eines Glycerincarbonats mittels wasserfreier Base beschrieben. Durch den Aufarbeitungsschritt wird der Glyceringehalt auf 3,5-4,1 Gew.-% gesenkt.

Mit der Aufarbeitung fällt neben dem zusätzlichen Arbeitsschritt auch ein Ausbeuteverlust an.

Aufgabe der Erfindung war es Glycerincarbonat in hoher Reinheit und mit hohen Ausbeuten herzustellen.

Die Aufgabe wurde gelöst durch ein Verfahren zur Herstellung von Glycerincarbonat, **dadurch gekennzeichnet, dass** Glycerin mit Dialkylcarbonaten oder cyclische Carbonaten in Gegenwart eines basischen Katalysators aus einer Mischung von mindestens zwei Komponenten aus den Gruppen der Metallhydroxide und -chloride, Metallhydroxide und -oxide, Metallchloride und -oxide umgeestert wird.

Überraschend wurde gefunden, dass das erhaltene Rohprodukt nur geringe Mengen an Glycerin enthält.

Es wurde gefunden, dass durch die hohe Reinheit des Rohproduktes, insbesondere der niedrige Gehalt an Glycerin, auf einen Aufreinigungsschritt verzichtet werden kann. Damit kann ein Verfahrensschritt gespart werden und zudem die Ausbeute erhöht werden.

Als Ausgangsstoffe werden Dialkylcarbonate, bevorzugt Dimethylcarbonat bzw. Diethylcarbonat eingesetzt.

Außerdem können cyclische Carbonate, vorzugsweise Ethylencarbonat verwendet werden.

Der basische Katalysator bzw. das Katalysatorgemisch wird in Mengen von 0,01 bis 10 Gew.-% eingesetzt.

Als basische Katalysatoren aus der Gruppe der Alkali- oder Erdalkalihydroxide, -chloride und/oder -oxide werden bevorzugt Mischungen aus 2 oder 3 Komponenten aus der Gruppe der Lithiumhydroxide oder Kaliumhydroxide oder Calciumhydroxide und/oder Lithiumchloride oder Kaliumchloride oder Calciumchloride und/oder Calciumoxide eingesetzt.

Bevorzugt werden Mischungsverhältnisse von Hydroxiden zu Oxiden von 1 zu 99% bis 99 zu 1%, besonders bevorzugt 15 zu 85% bis 30 zu 70%, vorzugsweise bei LiOH : CaO von 28 zu 72%, eingesetzt.

Bevorzugte Mischungsverhältnisse für Chloride mit Hydroxiden liegen bei 1 zu 99% bis 99 zu 1 %, besonders bevorzugt bei 15 zu 85% bis 30 zu 70%, vorzugsweise für LiCl: Ca(OH)₂ bei 20 zu 80%.

Bevorzugte Mischungsverhältnisse für Chloride mit Oxiden liegen bei 1 zu 99% bis 99 zu 1 %, besonders bevorzugt bei 15 zu 85% bis 30 zu 70%, vorzugsweise für LiCl: CaO bei 20 zu 80%.

Die Umesterung erfolgt bei Temperaturen von 60 - 150°C, vorzugsweise bei 70-85°C.

Die Umesterung erfolgt vorzugsweise unter Normaldruck. Versuche der Umesterung im Vakuum waren jedoch auch erfolgreich. Ebenso kann unter erhöhtem Druck gearbeitet werden.

Verwendungsgebiete für Glycerincarbonat hergestellt nach dem erfindungsgemäßen Verfahren sind beispielsweise Vorprodukt für Lacke und Klebstoffe.

Die im Folgenden gegebenen Beispiele werden zur besseren Veranschaulichung der vorliegenden Erfindung gegeben, sind jedoch nicht dazu geeignet, die Erfindung auf die hierin offenbarten Merkmale zu beschränken.

### Beispiele

### Beispiel 1 (B1):

| | | |
|---|---|---|
| 46,1 g | Glycerin wasserfrei Fa. Merck | = 0,5 mol |
| 180,2 g | Dimethylcarbonat Fa. Merck | = 2,0 mol |
| 1,1 g | Mischkatalysator (28% LiOH / 72% CaO) | = 0,5 % bez. a. Ansatz |

Der Ansatz wird in die Reaktionsapparatur eingefüllt und 9 Stunden unter Rückfluss erhitzt. Nach dem Abkühlen wird der Katalysator durch Filtration abgetrennt: Die Niedersieder werden danach am Rotationsverdampfer im Vakuum abdestilliert, anschließend wird nochmals filtriert und das Rohprodukt erhalten.

### Beispiel 2 (B2):

| | | |
|---|---|---|
| 276,6 g | Glycerin wasserfrei Fa. Merck | = 3,0 mol |
| | 1081,2g Dimethylcarbonat Fa. Merck | = 6,0mol |
| 6,6 g | Mischkatalysator (28% LiOH / 72% CaO) | = 0,5 % bez. a. Ansatz |

Der Ansatz wird in die Reaktionsapparatur eingefüllt und 8 Stunden unter Rückfluss erhitzt. Nach dem Abkühlen wird der Katalysator durch Filtration abgetrennt. Die Niedersieder werden danach am Rotationsverdampfer im Vakuum abdestilliert, anschließend wird nochmals filtriert und das Rohprodukt erhalten.

### Beispiel 3 (B3):

| | | |
|---|---|---|
| 46,1 g | Glycerin wasserfrei Fa. Merck = | 0,5mol |
| 180,2 g | Dimethylcarbonat Fa. Merck | = 2,0 mol |
| 1,1 g | Mischkatalysator (20% LiCl / 80% Ca(OH)₂) | = 0,5% bez. a. Ansatz |

Der Ansatz wird in die Reaktionsapparatur eingefüllt und 8 Stunden unter Rückfluss erhitzt. Nach dem Abkühlen wird der Katalysator durch Filtration abgetrennt. Die Niedersieder werden danach am Rotationsverdampfer im Vakuum abdestilliert, anschließend wird nochmals filtriert und das Rohprodukt erhalten.

### Beispiel 4 (B4):

| | | |
|---|---|---|
| 46,1 g | Glycerin wasserfrei Fa. Merck | = 0,5mol |
| 180,2 g | Dimethylcarbonat Fa. Merck | = 2,0 mol |
| 1,1 g | Mischkatalysator (20% LiCl / 80% CaO) | = 0,5 % bez. a. Ansatz |

Der Ansatz wird in die Reaktionsapparatur eingefüllt und 8 Stunden unter Rückfluss erhitzt. Nach dem Abkühlen wird der Katalysator durch Filtration abgetrennt. Die Niedersieder werden danach am Rotationsverdampfer im Vakuum abdestilliert, anschließend wird nochmals filtriert und das Rohprodukt erhalten.

**Versuche zur Herstellung von Glycerincarbonat**

| **Versuch-Nr.** | **Katalysator % bez. a. Ans.** | **Glycerin** | **DMC*** | **Reaktion** | | | | | **Produkt FI%** | **Masse Aussehen** |
|---|---|---|---|---|---|---|---|---|---|---|
| | | **mol** | **mol** | **Temp.** **C** | **Zeit** **h** | **Glycerin** **FI%** | **DMC** **FI%** | **GMDC*** | | |
| B1 | Li-Kalk: LiOH / CaO (28% / 72%) 0.5% | 0,5 | 2 | 80-76 | 9 | 0,20 | - | 2,6 | 96,1 | 60g farblos, klar |
| B2 | Li-Kalk: LiOH / CaO (28% / 72%) 0.5% | 3,0 | 12,0 | 77-73 | 8 | 0,05 | - | 2,0 | 95,8 | 354g farblos, klar |
| B3 | Modifizierter Li-Kalk LiCl / Ca(OH)2 (20% / 80%) 0,005 | 0,5 | 2 | 80-74 | 8 | 0,80 | - | 2,2 | 95,2 | 59g farblos, klar |
| B4 | LiCl / CaO (20% / 80%) 0,005 | 0,5 | 2 | 80-74 | 8 | 0,14 | - | 0,5 | 97 | 56g farblos, klar |

| | | | | | | | | | | |
|---|---|---|---|---|---|---|---|---|---|---|
| DMC * = Dimethylcarbonat *Weitere Peaks nicht berücksichtigt GMDC = Glycerinmethyldicarbonat n. b. = nicht bestimmt | | | | | | | | | | |

Die Beispiele B1, B2, B3 und B4 zeigen hohe Produktausbeuten und sehr geringe Glycerinanteile im Produkt. Beispiel 4 zeigt zusätzlich eine geringe Konzentration an Glycerinmethyldicarbonat.

## Patentansprüche

1. Verfahren zur Herstellung von Glycerincarbonat, **dadurch gekennzeichnet, dass** Dialkylcarbonate oder cyclische Carbonate in Gegenwart eines basischen Katalysators aus einer Mischung von mindestens zwei Komponenten aus den Gruppen der Metallhydroxide und -chloride, Metallhydroxide und -oxide, Metallchloride und -oxide umgeestert werden.

2. Verfahren zur Herstellung von Glycerincarbonat nach Anspruch 1, **dadurch gekennzeichnet, dass** in Gegenwart von Alkali- oder Erdalkalihydroxiden, - oxiden und/oder -chloriden umgeestert wird.

3. Verfahren zur Herstellung von Glycerincarbonat nach Anspruch 1, **dadurch gekennzeichnet, dass** in Gegenwart von LiCl/CaO umgeestert wird.

4. Verfahren zur Herstellung von Glycerincarbonat nach Anspruch 1, **dadurch gekennzeichnet, dass** der Katalysator in Mengen von 0,01 bis 10 Gew.-% eingesetzt wird.

5. Verfahren zur Herstellung von Glycerincarbonat nach Anspruch 1, **dadurch gekennzeichnet, dass** Katalysatormischungen im Verhältnis von 1 zu 99% bis 99 zu 1% eingesetzt werden.

6. Verfahren zur Herstellung von Glycerincarbonat nach Anspruch 1, **dadurch gekennzeichnet, dass** die Umesterung bei 60-150°C erfolgt.

7. Verfahren zur Herstellung von Glycerincarbonat nach Anspruch 1, **dadurch gekennzeichnet, dass** die Umesterung unter Normaldruck oder im Vakuum erfolgt.

8. Verwendung von Glycerincarbonat hergestellt gemäß Anspruch 1 als Vorprodukt für Lacke und Klebstoffe.

## Claims

1. Process for preparing glyceryl carbonate, **characterized in that** dialkyl carbonates or cyclic carbonates are transesterified in the presence of a basic catalyst composed of a mixture of at least two components from the groups of the metal hydroxides and chlorides, metal hydroxides and oxides, metal chlorides and oxides.

2. Process for preparing glyceryl carbonate according to Claim 1, **characterized in that** transesterification is effected in the presence of alkali metal or alkaline earth metal hydroxides, oxides and/or chlorides.

3. Process for preparing glyceryl carbonate according to Claim 1, **characterized in that** transesterification is effected in the presence of LiCl/CaO.

4. Process for preparing glyceryl carbonate according to Claim 1, **characterized in that** the catalyst is used in amounts of 0.01 to 10% by weight.

5. Process for preparing glyceryl carbonate according to Claim 1, **characterized in that** catalyst mixtures in a ratio of 1:99% to 99:1% are used.

6. Process for preparing glyceryl carbonate according to Claim 1, **characterized in that** the transesterification is effected at 60-150°C.

7. Process for preparing glyceryl carbonate according to Claim 1, **characterized in that** the transesterification is effected under standard pressure or under reduced pressure.

8. Use of glyceryl carbonate prepared according to Claim 1 as a precursor for coatings and adhesives.

## Revendications

1. Procédé pour la préparation de carbonate de glycérol, **caractérisé en ce qu'**on soumet à une transestérification des carbonates ou carbonates cycliques de dialkyle en présence d'un catalyseur basique à base d'un mélange d'au moins deux composants choisis dans les groupes des hydroxydes et chlorures de métaux, des hydroxydes et oxydes de métaux, des chlorures et oxydes de métaux.

2. Procédé pour la préparation de carbonate de glycérol selon la revendication 1, **caractérisé en ce qu'**on effectue la transestérification en présence d'hydroxydes, d'oxydes et/ou de chlorures de métaux alcalins ou alcalino-terreux.

3. Procédé pour la préparation de carbonate de glycérol selon la revendication 1, **caractérisé en ce qu'**on effectue la transestérification en présence de LiCl/CaO.

4. Procédé pour la préparation de carbonate de glycérol selon la revendication 1, **caractérisé en ce qu'**on utilise le catalyseur en quantités de 0,01 à 10 % en poids.

5. Procédé pour la préparation de carbonate de glycérol selon la revendication 1, **caractérisé en ce qu'**on utilise des mélanges de catalyseurs en un rapport de 1:99 % à 99:1 %.

6. Procédé pour la préparation de carbonate de glycérol selon la revendication 1, **caractérisé en ce qu'**on effectue la transestérification à 60-150 °C.

7. Procédé pour la préparation de carbonate de glycérol selon la revendication 1, **caractérisé en ce qu'**on effectue la transestérification sous la pression normale ou sous vide.

8. Utilisation du carbonate de glycérol préparé selon la revendication 1, en tant que produit intermédiaire pour des peintures et des adhésifs.
